(19) Europäisches Patentamt / European Patent Office / Office européen des brevets

(11) **EP 2 205 982 B1**

(12) **EUROPEAN PATENT SPECIFICATION**

(45) Date of publication and mention
of the grant of the patent:
**14.12.2011 Bulletin 2011/50**

(51) Int Cl.:
*G01P 5/26* (2006.01)    *G01P 3/36* (2006.01)
*G01S 17/58* (2006.01)   *G01S 7/481* (2006.01)
*G01F 1/66* (2006.01)    *A61B 5/026* (2006.01)
*G01J 1/44* (2006.01)    *H04N 5/335* (2011.01)

(21) Application number: **08843697.7**

(22) Date of filing: **30.10.2008**

(86) International application number:
**PCT/GB2008/003660**

(87) International publication number:
**WO 2009/056825 (07.05.2009 Gazette 2009/19)**

(54) **APPARATUS AND METHODS FOR IMAGING**

BILDGEBENDE VORRICHTUNGEN UND VERFAHREN

APPAREILS ET PROCÉDÉS D'IMAGERIE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.10.2007 GB 0721165**

(43) Date of publication of application:
**14.07.2010 Bulletin 2010/28**

(73) Proprietor: **The University of Nottingham Nottingham NG7 2RD (GB)**

(72) Inventors:
 • **MORGAN, Stephen**
   **Nottingham NG7 2RD (GB)**
 • **HAYES-GILL, Barrie**
   **Nottingham NG7 2RD (GB)**
 • **CROWE, John**
   **Nottingham NG7 2RD (GB)**
 • **WOOLFSON, Malcolm**
   **Nottingham NG7 2RD (GB)**

(74) Representative: **Swindell & Pearson Limited**
   **48 Friar Gate**
   **Derby**
   **DE1 1GY (GB)**

(56) References cited:
   **EP-A- 1 332 718    JP-A- 10 221 159**

 • **DMOCHOWSKI P R ET AL: "Camera pixel for coherent detection of modulated light" ELECTRONICS LETTERS, IEE STEVENAGE, GB, vol. 40, no. 22, 28 October 2004 (2004-10-28), pages 1403-1404, XP006022809 ISSN: 0013-5194**

**Description**

The present invention relates to apparatus and methods for imaging, and in particular, but not exclusively, to apparatus and methods for imaging flows and vibrations.

The measuring and imaging of flows and vibrations is important in many applications. For example, flow applications include air flow measurements in aerodynamic design, fluid flow in pipes, mixing in combustion engines, mixing of supercritical fluids in pharmaceutical production, formation of tissue engineered structures and the parallel monitoring of traffic flow. Imaging of vibration is important in many mechanical design applications as artificial identification systems, for civil engineering structures, aircraft fuselage integrity, engine housing assessment, automobile vibrations to name but a few.

DMOCHOWSKI et al: "Camera pixel for coherent detection of modulated light" ELECTRONICS LETTERS, IEE STEVENAGE, GB, vol. 40, no. 22, 28th October 2004 (2004 - 10 - 28), pages 1403-1404, XP006022809 ISSN : 0013-5194 describes apparatus comprising an electromagnetic radiation sensor for producing an output when illuminated by modulated light. A sensor element produces a photocurrent which passes through a transistor.

EP-A-1 332 718 (Stichting) describes laser Doppler perfusion imaging using a CMDS image sensor.

JP 10 221159 A (Tokyo) describes a laser Doppler type vibration distribution measuring apparatus.

In a first aspect of the invention, example embodiments provide apparatus comprising:

at least one electromagnetic radiation sensor for producing an output when illuminated by modulated laser light;

the sensor, in use, providing an output determined by a laser Doppler signal illuminating the sensor;

and wherein the sensor comprises a photoelectric sensor element (34) producing a photocurrent (I) when illuminated, and a transistor (36) through which the photocurrent passes, characterised in that the sensor, in use, provides an output determined by a laser Doppler signal illuminating the sensor; and in that the transistor operates in a sub threshold region to provide a DC output voltage which is a logarithmic function of the DC current, and in that the transistor provides an AC sensor output from the photocurrent when the sensor element is illuminated by a laser Doppler signal, the AC output being normalised by the detected DC level.

In this aspect of the invention, example embodiments also provide method comprising:

providing at least one electromagnetic radiation sensor for producing an output when illuminated by modulated laser light;

and wherein the sensor comprises a photoelectric sensor element (34) producing a photocurrent (I) when illuminated, and a transistor (36) through which the photocurrent passes, characterised in that the sensor, in use, provides an output determined by a laser Doppler signal illuminating the sensor; and in that the transistor operates in a sub threshold region to provide a DC output voltage which is a logarithmic function of the DC current, and in that the transistor provides an AC sensor output from the photocurrent when the sensor element is illuminated by a laser Doppler signal, the AC output being normalised by the detected DC level.

Additional features of each of these aspects of the invention are set out in the attached claims, to which reference should now be made.

Embodiments of the present invention as well as examples of sensors not being embodiments will now be described in more detail, with reference to the accompanying drawings, in which:

Fig. 1 illustrates an illumination configuration;

Fig. 2 is a block diagram of sensor output processing arrangements;

Figs. 3a and 3b illustrate pixel circuits having a logarithmic output;

Figs. 4a to 4c illustrate responses of an HDA amplifier;

Figs. 5a to 5d illustrate circuits and responses for filters based on operational transconductance amplifiers;

Figs. 6a and 6b illustrate statistics associated with example methods of the invention;

Figs. 7a and 7b are block diagrams of example circuits of the invention;

Figs. 8 and 9 illustrate practical implementations of example devices of the invention; and

Figs. 10a and 10b illustrate a normalising pixel and its performance.

The examples to be described relate to systems for the imaging of flows and vibrations. The examples involve the illumination of a subject by electromagnetic radiation (ER). The electromagnetic radiation may be visible light or may be at an invisible wavelength. In this specification, the terms electromagnetic radiation, ER and "light" are used interchangeably and none is intended to be restricted to visible light or invisible wavelengths.

ER (for example visible light but not limited to these frequencies) that interacts with a moving object undergoes a Doppler shift (i.e. a change in frequency) that is proportional to the velocity of the object. The change of frequency of an ER wave when it interacts with a moving object is the well-known Doppler effect. The frequency shift of the detected wave can be related to the velocity of the moving object;

$$\Delta f = \frac{2nv}{\lambda} \cos \vartheta$$

Where $\Delta f$ is the Doppler frequency shift, n is the refractive index, v is the velocity of the target, $\lambda$ is the wavelength of the illumination and $\vartheta$ is the angle between the illumination and the axis of motion. It should be noted that when the motion of the object is perpendicular to the illumination then no Doppler shift will occur.

Several approaches have been developed to address the problem of measuring flows and vibrations. Laser Doppler anemometry [1,2] has been successfully applied for many years to make *single point* measurements. Although these single point systems are capable of measuring rapidly changing flows the obvious disadvantage is that flows that vary spatially cannot be imaged without the addition of scanning. For example, imaging would provide an understanding of the flow of air around an automobile, or the mixing process in a combustion engine. The same applies to vibrometry where single point measurements have been common practice for many years and commercial systems exist. The ability to provide a full field spatial map of the flow or vibration profile provides considerable additional information.

Scanning using a single point system configuration allows an image to be built up pixel by pixel [3,4]. However, this approach is limited by the scanning rate and so does not provide real time imaging of flows and cannot provide information about rapidly changing processes.

Given the importance of rapidly imaging flows, other techniques have been developed. The most notable of these are particle image velocimetry (PIV) and Doppler global velocimetry (DGV) [5,6]. PIV is a time of flight method based on measuring particle displacement between two sequential images, separated by a known time interval. The displacements can then be determined by image correlation. The technique has found widespread use but there are some drawbacks[5];

(i) It is necessary to produce optics that can distinguish between individual seeding particles. These particles then need to be imaged twice.
(ii) Considerable off line signal processing is then required to deconvolve the two images into a velocity map.
(iii) 3D velocity information is difficult to achieve.
(iv) The spatial resolution is reduced by the need to correlate sub-images.

To overcome these problems researchers have been developing DGV. This technique uses conventional CCD cameras and molecular filters that absorb at the frequency of the incident light. Light that undergoes a Doppler shift is attenuated by different amounts by the molecular filter and by calibrating the intensity of the detected light the flow profile can be imaged. This technique shows promise for single shot, 3D whole field velocity determination. The processing is similar and therefore can be performed in real time. It is not necessary to image individual particles and so the requirements for the imaging optics are much less stringent. The spatial resolution is also higher as flow can be measured at each pixel. There are some disadvantages as the system requires accurate calibration of the laser, iodine cell, and alignment of signal and reference images. This results in the flow resolution not being as high as that of single point laser Doppler systems.

In vibrometry measurements, Aguanno *et al* [7,8] have used a commercial CMOS-DSP camera (Fastcom Technology, Switzerland) consisting of a CMOS photodiode array with an independent digital signal processing unit to image vibrations without mechanical scanning using the Doppler approach. The CMOS-DSP camera has a data bottleneck as the data needs to be transferred from the imaging array to the off chip processor which limits the frequencies that can be imaged (for a 64 by 64 pixel array the sampling rate is 500 Hz). Again, this compromises the performance of the system with respect to either or both the maximum detectable frequency or image pixel resolution.

Kimachi and Ando [9] developed a CMOS camera and reported its use to detect the 2-D vibrations produced by a loud speaker. A structured (bright and dark fringes), modulated light source was used to illuminate the surface and the system contained no separate optical reference. The relative change of the fringe pattern to an electrical local oscillator signal was used to measure the vibration of the surface. The camera comprised a 64x64-pixel array with a time domain correlator at each pixel. This gave the time domain correlation of a detected modulated light signal with an electrical local oscillator signal. This approach however, is not a Laser Doppler based method.

Other approaches exist to image flow and vibrations include Electronic Speckle Pattern Interferometry (ESPI) [10] and holographic imaging [11]. However these are not closely related to the embodiment described here. Phase Doppler Velocimetry [12] is a similar approach to Laser Doppler Velocimetry in which the phase difference of the Doppler signals between two detectors positioned at different angles is used to provide an estimate of the size of moving particles.

Integrated optical sensors (IOS) are arrays of photodiodes with on-chip processing. Image sensors fabricated from MOS integrated circuit technology first entered the market in the early 1960's with the use of passive photodetectors and subsequently active pixel sensors (APS). The quality of these early devices was generally poor and they were soon replaced by CCD technology, which currently dominates the market. Although CCDs exhibit excellent performance they suffer from the fact that specialist fabrication techniques are required that are incompatible with standard CMOS processes. This limits the amount of on-chip circuitry possible and leads to a bottleneck where the data is serialised off chip to an external processor. Integrated optical sensors offer tremendous advantages in terms of speed, signal to noise and dynamic range, data compression and throughput, optical geometry, size and weight and scalability.

We also define an IOS detector in its widest Electromagnetic Radiation (ER) sense not limited to visible light. For example a hybrid arrangement is possible of an array of ER detectors "bump bonded" at the pixel level to an array of parallel processing elements. Such an arrangement is useful for using detectors other than silicon and hence a different wavelength range of ER is possible i.e. using HgCdTe for wavelengths around ~1.3□m. This allows the ability of the detector to use eye safe radiation and takes full advantage of the Integrated Circuit processing for the generation of.an array of parallel processing pixels. Such an approach can also be extended to much lower and higher frequencies and even to non-ER modulation, for example an array of ultrasound detectors can be bump bonded to an Integrated circuit with processing at each detector point.

Morgan and Hayes-Gill [13] have described an apparatus that combines an integrated optical sensor with appropriate on-chip signal processing with an ER configuration for detecting Doppler shifts, a system for imaging flows and vibrations in full-field can be obtained. The on-chip signal processing is capable of extracting in parallel useful information, e.g. flow, vibration, velocity, etc from the high frequency oscillating signals detected. The useful information is then readout from the sensor at a lower data rate. This allows full field measurements of flows at high resolution, even when the Doppler signals are oscillating at high frequency.

Laser Doppler blood flow imaging has followed a similar development route to the imaging of flows and vibrations. Scanning systems have been developed [14] but the image acquisition rate is slow and these are sensitive to patient movement artefacts. The main difference between blood flow imaging and those systems imaging other flows and vibrations is that there is no separate reference beam for blood flow imaging as the reference comes from static tissue.

Full field imaging of blood flow has been developed in the form of the laser speckle contrast analysis (LASCA) [15]. This approach uses the reduction in contrast caused by a fluctuating speckle pattern to measure the blood flow but is not a Doppler approach.

An apparatus for the Doppler imaging blood flow (limited to the wavelengths only suitable for silicon detectors) based on a commercial photodiode array [16] (Boggett) and a fast read out rate CMOS camera have been described [17] (Serov). A fast read out rate sensor is used which means that the image data has to be transferred at high speed to an external processor. This system is limited by the data transfer bottleneck from the sensor array to a DSP (digital signal processor). This compromises the performance of the system with respect to either or both the maximum detectable frequency or image pixel resolution.

Serov [17] has mentioned the possibility of including an on-chip digital signal processor but no details have been provided as to how this will be achieved. This is not straightforward as the performance needs to be optimized with respect to silicon area so that the mark space ratio of the imager remains high and the sensors can be fabricated at a comparatively low cost. The signals are typically of low modulation depth (~1-10%) and are at low frequency (a few tens of Hz up to ~20KHz). Practically, one would not simply duplicate the off-chip processing on-chip and innovative steps are required in the design and fabrication of integrated optical sensors.

## *Illumination Configuration*

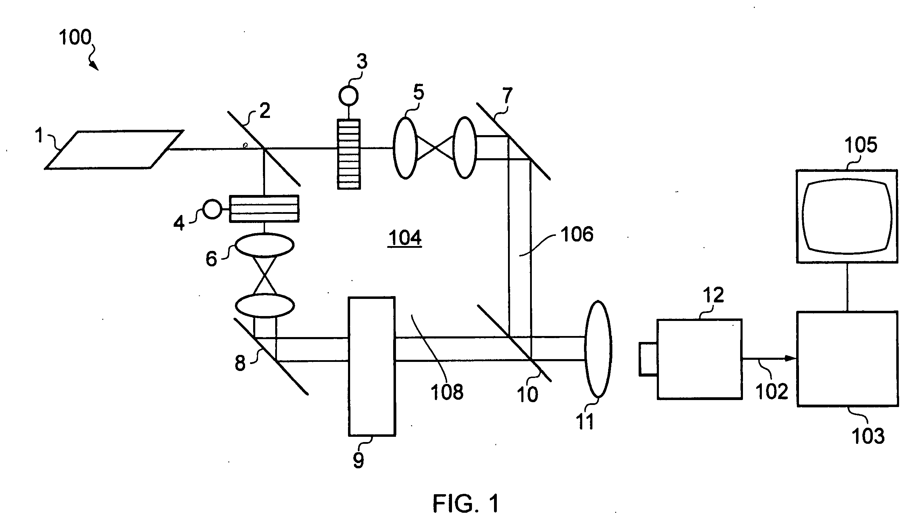

Fig 1 illustrates a system 100 comprising a sensor device 12 providing an array of electromagnetic radiation sensors (to be described in more detail). The system 100 also includes processing circuitry integrated in the sensor device 12 and operable to process the output of the sensors to provide the output of the sensor device, at 102.

An illumination system 104 is operable to illuminate the sensors with a reference beam 106 of electromagnetic radiation and with electromagnetic radiation 108 reflected (in this example) from a subject (or target) 9.

The processing circuitry integrated in the sensor device 12 is operable to provide, for each operative sensor, a value calculated from the Doppler shift of the reflected radiation at the corresponding position at the subject 9, and to provide the calculated values as the processed output 102.

One example configuration using for example ER in the optical part of the electromagnetic spectrum is shown in figure 1. Light emerging from a laser (1) is split using a beamsplitter (2) into a probe beam 108 that interacts with the target (9) and a reference beam 106. In this example, both beams 106, 108 are passed through a component (3,4) that shifts the frequency of the light e.g. an acousto-optic modulator, but this is not essential. Frequency shifting the light enables the direction of the Doppler shift (flow direction) to be determined. Both beams are then expanded by a combination of lenses (5,6) to provide full field illumination (i.e. illumination of an area rather than a single point). The probe beam 108 then interacts with the target (9) and is frequency shifted by any motion of the target. The probe beam 108 and reference beam 106 are then recombined by a pair of mirrors (7,8) and a beamsplitter (10). The object is imaged onto an area of the integrated optical sensor (12) using an imaging lens (11) to provide a modulated light signal proportional to the velocity of the subject 9. This modulated light signal, eliminating the sensor, is here called the laser Doppler signal. It should be noted that when the motion of the subject 9 is perpendicular to the illumination 108, then no Doppler shift will occur.

It is to be understood that many alternative illumination configurations can be devised, for example utilising reflectance or transmission geometries.

The example sensors to be described detect and process laser doppler signals and efficiently use silicon area by a series of innovative steps which can be used on their own or optimally combined together. Each of these steps will now be described individually.

The main components in processing laser Doppler signals are normalization, amplification, filtering and square and average. Normalization is required to compensate for fluctuations in laser power and skin reflectance. Band-pass and frequency weighted (by $\omega^{0.5}$) filtering are required to calculate the blood concentration and blood flow respectively.

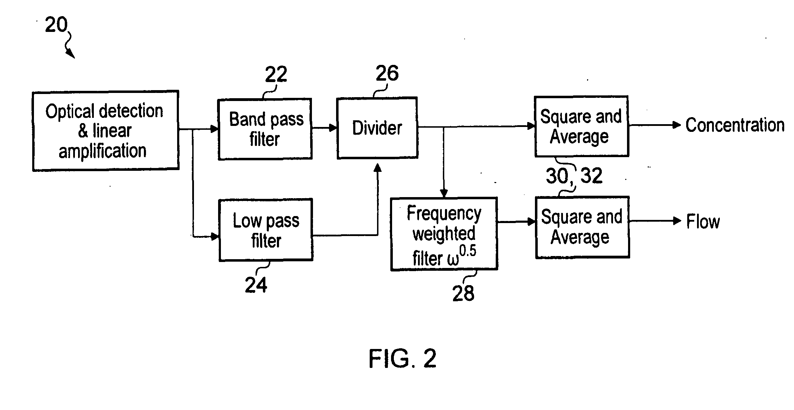

The standard processing electronics have been previously described many times but are shown in figure 2 [18]. The signals are amplified by a linear front-end 20 (photodiode + transimpedance amplifier) and then the AC and DC components are separated at 22, 24 so that normalization can be performed by a divider 26. The normalized signal then passes to a band-pass filter, followed by a frequency weighted filter 28. Squaring and averaging 30, 32 reduces noise. Depending on the choice of the designer, the signal can be digitized (using an analogue to digital converter) at different points in the processing so that either analogue or digital signal processing can be utilized.

***Normalization with a logarithmic pixel;*** (embodiment of the invention)

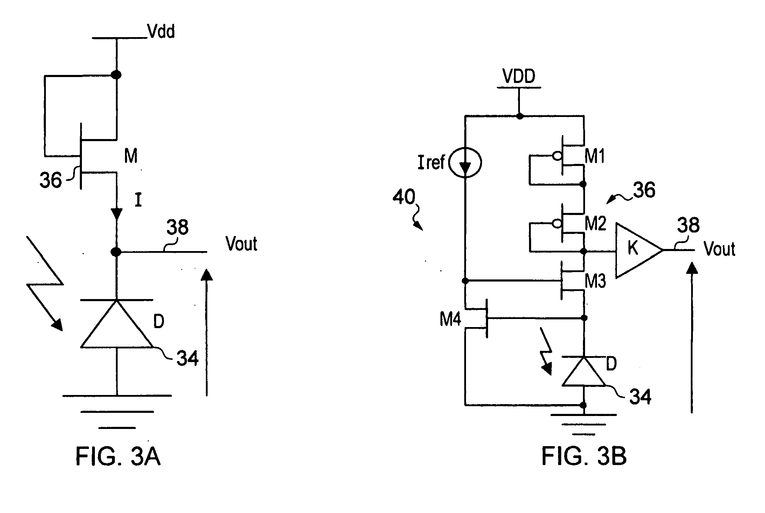

Instead of the standard linear front-end a logarithmic front-end is used. That is, the output of the sensor is a logarithmic function of the illuminating laser Doppler signal. The sensor comprises a photoelectric sensor element 34 producing a photocurrent when illuminated, and a transistor 36 through which the photocurrent passes, and which operates in a sub threshold region to provide a sensor output 38 from the photocurrent. This circuit can be configured as either without (fig. 3a) or with feedback 40 (fig. 3b). At electrical photocurrent levels usually obtained in laser Doppler imaging, the MOS transistors operate in the subthreshold region [19,20] where the output DC voltage is a logarithmic function of the DC current.

$$I_{DC(subthreshold)} = I_o \exp(n'qV_{GS}/kT)$$

Where n' is the subthreshold slope factor and depends on the number of diode connected loads.

In this embodiment the sensor element 34 is in series with the channel of the transistor 36, the output 38 being taken at the connection between the sensor element and the transistor. The photoelectric sensor element is a photodiode. The transistor is an MOS transistor in this example, and the output is an output voltage.

Now, since $V_{GS} = V_{DS} = V_{DD} - V_{out}$

And rearranging we get:

$$V_{out} = V_{DD} - n'U_t \ln I_{DC} + n'U_t \ln I_o$$

Where $U_t = kT/q$

From the DC I-V relationship, the transimpedance (which is also the AC gain of the TIA) of a single transistor TIA can be derived as:

$$\text{Gain} = dV_{out}/dI_{DC} = n'U_t/I_{DC} \tag{1}$$

where $g_m$ is the transconductance of the transistor, n' is the subthreshold slope factor, $U_T$ is the thermal voltage (25.8 mV at room temperature) and $I_{DC}$ is the DC photocurrent. Since the AC Doppler component is directly proportional to the DC component striking the object (and reflected from the object), one can define:

$$I_{DC} = m \cdot i_{ac} \tag{2}$$

Where $i_{ac}$ is the amplitude of the AC current and m is the Doppler ratio whose typical value in blood flowmetry ranges from 10 to 100. The output AC voltage can be obtained as:

$$v_{ac} = i_{ac} \cdot \frac{1}{g_m} = \frac{I_{DC}}{m} \frac{n'U_T}{I_{DC}} = \frac{n'}{m} U_T \tag{3}$$

Equation (3) shows that the output AC voltage is determined by the subthreshold slope factor (n'), thermal voltage ($U_T$) and Doppler ratio (m). Therefore, for a given Doppler ratio, any fluctuations in the laser source power output ($I_{DC}$) or variations in skin remittance (also proportional to $I_{DC}$) will not affect the output AC signal, thus providing normalization at the pixel level. This removes the need for a separate DC channel (low pass filter) and a divider on-chip thus providing an efficient reduction in silicon area.

Accordingly, it can be seen that in this example embodiment, the sensor is operable to provide a normalised output based upon the DC input light level. The sensor provides an AC output.

In this example, the sensor is a semiconductor device. The transistor can be integrated into the semiconductor device in order to provide on chip processing with efficient use of silicon.

### Normalization with an integrating pixel and digital counter;

An alternative to the analogue logarithmic pixel is to use an integrating pixel with a comparator and a counter[28]. This circuit performs:

- Light conversion into a voltage
- Conversion into a digital representation. That is, the sensor element output may be digitised prior to normalisation.
- Normalization

Accordingly, in this example, the output of the sensor is normalised. That is, the sensor element provides an output, and a normalising circuit operates to normalise the output of the sensor element, to provide a sensor output. The sensor element and the normalising circuit may both be semiconductor devices, so that the normalising circuit may be integrated into the semiconductor device in order to provide on chip processing with efficient use of silicon.

Such a pixel is also a very useful alternative for Doppler since it both normalizes and digitizes by using a very efficient digital counter.

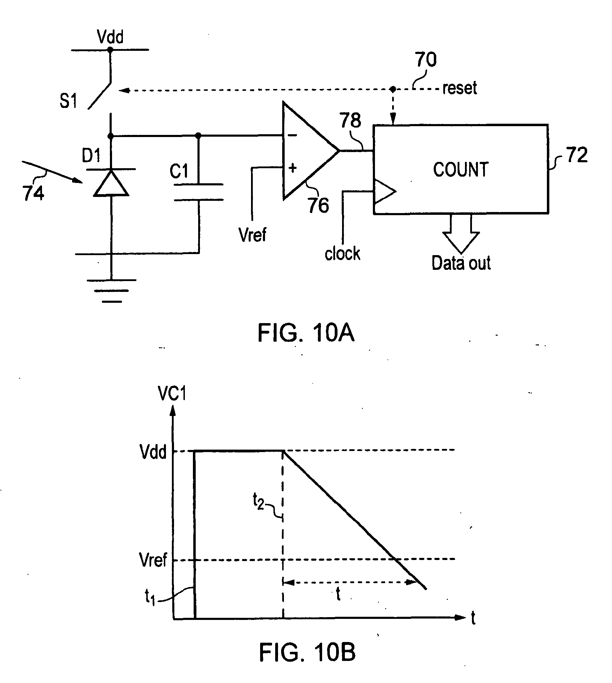

A typical integrating pixel circuit with digitization is shown in Figures 10(a) and 10(b).

The pixel works as follows. The switch "S1" is controlled by the "reset" signal 70 such that when the reset 70 is enabled (time $t_1$ in Fig. 10(b)) the switch "S1" closes and the voltage on the photodiode, D1, is pulled up to the supply voltage Vdd which therefore fully charges capacitor C1. The capacitance of C1 can be provided either by the inherent capacitance of the photodiode D1 or by an additional capacitor. Additionally, when the "reset" 70 is enabled the digital counter 72 is reset to zero.

When, the "reset" line 70 is disabled (time $t_2$ in Fig. 10b)), the digital counter 72 starts counting clock pulses received at 73 and the switch S1 opens. Assuming that switch S1 is an ideal switch, then the voltage on the capacitor C1 is slowly discharged through the photodiode D1 at a rate determined by the level of light/electromagnetic radiation 74 on the photodiode and the magnitude of the capacitance C1. The relationship between the current in the photodiode D1 and the rate of discharge follows the standard capacitor discharge equation:

$$I = C1\frac{dV_{C1}}{dt} \dots Equation\ I1$$

The voltage on the discharging capacitor C1 is also connected to the inverting input of an analogue comparator 76 whose value is compared to a preset reference level, "Vref". When the discharge (capacitor) voltage drops below "Vref" the "stop" signal 78 is enabled and the counter 72 is stopped. The resulting digital count of clock pulses 73 directly represents the time taken to discharge from Vdd to Vref - labelled as "t" in Figure 10b. By rearranging Equation 11 we can see that this pixel performs integration:

$$t = \frac{C1}{I} \int dV_{C1} \dots Equation\ I2$$

From Equation I1 we can see that if the light level increases (so that the photocurrent I through the diode also increases) then the rate of discharge will also increase and from Equation I2 the value of "t" will decrease. We therefore have a relationship that shows that "t" is proportional to the reciprocal of the DC light level as shown in Figure 10b.

In this application we are interested in the Doppler generated wave i.e we have a modulated or AC signal. With a Doppler signal the modulation depth (AC component divided by the DC component) remains constant irrelevant of the DC light level i.e. if the DC light level increases by a factor of 3 then the AC component will also increase by the same magnitude. However, as a result of the reciprocal relationship the modulated digital representation results in a normalization effect on the Doppler signal.

### *Hysteretic differentiator amplifier to increase modulation depth of signals;*

as the modulation depth of signals in laser Doppler imaging is low (1-10%) generally a high number of bits are required when digitizing (typically at least 10 bits). This means that the on-chip analogue to digital converters (ADCs) will consume a greater chip area than if a smaller number of bits were required. The hysteretic differentiator amplifier (HDA) amplifies the AC component while leaving the DC component unaffected. This provides an increase in modulation depth of the signal and the opportunity to use a lower number of bits in the ADC, thus reducing the area of the on-chip processing.

That is, in this example, there is an amplifier arrangement operable to amplify the output of the sensor, the amplifier having a gain which is lower at DC than at the frequency range of the laser Doppler signal. The DC gain may be unity or less. In one example, again at the frequency range of the laser Doppler signal is at least 50 times the DC gain. This may be achieved by an amplifier arrangement whose gain has a cutoff frequency, below which the gain is lower. The cutoff frequency may be set in accordance with the expected Doppler signal frequencies from a subject to be image. That is, when the amplifier arrangement is designed, consideration can be made of the intended target (such as blood flow or another system), and the range of frequencies likely to be encountered within the laser Doppler signal returned from the intended target. This allows the cutoff frequency to be selected to be below the expected minimum Doppler signal frequency

The amplification of the AC Doppler signal on the chip is performed with for example a hysteretic differentiator amplifier (HDA) [21]. The HDA circuit consists of an operational transconductance amplifier (OTA) with an inverted CMOS inverter[29] and an NMOS transistor capacitor circuit in its feedback path.

The inverted CMOS inverter and the NMOS transistor capacitor circuit can be considered as an R-C circuit acting as a low pass filter in the feedback path of the amplifier where R and C are represented by the inverted CMOS inverter and NMOS transistor capacitor, respectively. The inverted CMOS inverter presents a very large resistance to the circuit and hence an extremely low cut-off frequency (~mHz) is produced. As a result only DC voltage (and very low frequencies) are fed back to the inverting input of the amplifier causing predominantly AC components of the signal at the non-inverting input of the amplifier to be amplified. This analog signal processing increases the modulation depth of the Doppler signal prior to digitising. It is not necessary to use this inverted CMOS inverter as the high resistance value of R since any other suitable passive high sheet resistance layer (i.e. very low conductivity or high sheet resistance material) or active component (e.g. HRES[22] or low $g_m$ circuit - see below) will suffice.

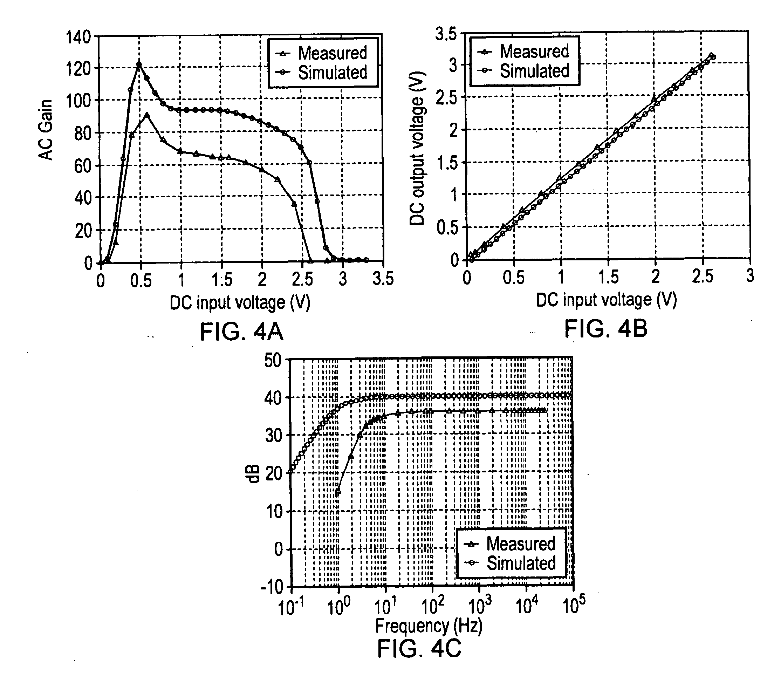

Figure 4 shows the measured and simulated responses of an on-chip HDA fabricated by our group. Any other amplifier that amplifies the AC and either holds the DC constant or removes the DC could of course be used.

The sensor element and the amplifier arrangement may both be semiconductor devices, so that the amplifier arrangement may be integrated into the semiconductor device in order to provide on chip processing with efficient use of silicon.

### Efficient filter design on-chip using operational transconductance amplifiers

In addition to improving the modulation depth of the detected signals, OTAs can also be used to implement the filters required for obtaining blood flow measurements. Small, compact versions of the band pass filter and frequency weighted filter are difficult to design on-chip due to the low frequencies required in some applications such as laser Doppler blood flowmetry. The large RC time constant requires large die area for resistors and/or capacitors, and the necessary accuracy of the RC time constant can not be achieved due to the variation in both the on-chip resistance and capacitance - a contributory factor for fixed pattern noise (FPN) [23,24].

The operational transconductance amplifier capacitor (OTA-C) filter [25] provides the opportunity to implement low frequency filters efficiently on-chip. The cut-off frequency of a basic OTA-C filter is determined by:

$$f_{cutoff} = \frac{g_m}{2\pi C} \tag{4}$$

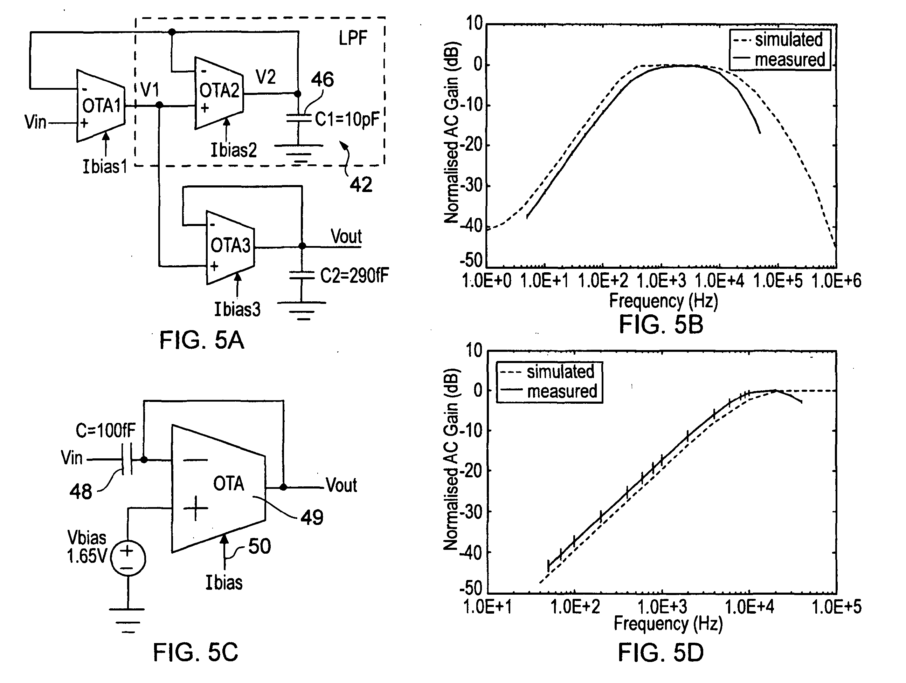

Where $g_m$ is the transconductance of the operational transconductance amplifier (OTA) and C is the capacitance which filters the output of the OTA. Since $g_m$ is controlled by the bias current through the OTA, the cut-off frequency can be controlled by this bias current. Figure 5a illustrates the HDA OTA-C band pass filter, which is of sufficiently small size that it can be fabricated at each pixel of the sensor. An OTA-C low pass filter 42, which is enclosed in the dash-dot box, consists of the operational transconductance amplifier labeled "OTA2" and the capacitor 46 "C1". Operational transconductance amplifier OTA1 can be treated as an all-pass device within the bandwidth of interest, which with the feedback from the LPF establishes a quasi high pass filter, known as a hysteretic differentiator amplifier (HDA). Here, we combine the HDA and the OTA-C to construct a HPF with both low cut-off frequency and high AC gain (by setting the gain of OTA1 high). To construct the band pass filter necessary for blood concentration measurements, a LPF (OTA3 and C2) is cascaded with the HPF.

The measured and simulated frequency response of an HDA OTA-C band pass filter fabricated by our group is shown in Fig. 5b. The frequency responses of four pixels along the diagonal of a 4x4 sensor array were measured to obtain the error bars. The simulated lower and upper cut-off frequencies are 250 Hz and 20 kHz respectively. The average of the measured lower cut-off frequency and upper cut-off frequency are 300 Hz and 15 kHz, respectively. The measured lower frequency cut-off is 50 Hz higher than simulated due to a parasitic capacitance in parallel with capacitor C1 and the actual leakage current of OTA2 being larger than the simulated leakage current. The measured upper cut-off frequency is 5 kHz lower than the simulated frequency due to the presence of an anti-aliasing filter ($f_{cutoff}$ = 20$kHz$) on the target printed circuited board (PCB).

Low pass filters can also be designed using this approach. This is unlikely to be necessary for extracting the DC for normalization, however, it may be required for obtaining a DC light image similar to a standard video image or offset noise calibration. A low pass filter can also be used as an anti-aliasing filter prior to digitization.

The GMC circuit shown in figure 5a [26] is used as an anti-aliasing filter before the signal is digitized by the ADC. It uses a single stage differential amplifier to provide the effective resistance of the circuit ($1/g_{m2}$) with an NMOS transistor providing the capacitance to form a low pass filter. The cut-off frequency of the GMC anti-aliasing filter can be controlled by an external bias current. In this case, the measured results also show that in order to achieve a 20KHz cut-off frequency from the GMC to avoid aliasing at the 40KHz sampling frequency usually required for laser Doppler blood measurements, the bias current of the GMC must be set at approximately 40$\mu$A. However, the use of source degeneration and multiple parallel differential transistors can significantly reduce this value.

The -3dB cut-off frequency of the filter is given by:

$$f_{-3dB} = \frac{g_{m2}}{2\pi C} \qquad (5)$$

where: $g_{m2}$ is the overall transconductance of the differential amplifier and C is the gate capacitance of the NMOS transistor. A useful feature of this filter is that the value of $g_{m2}$ and hence the bandwidth can be controlled by an external bias current.

It can be understood from the above description that in this example, the sensor is a semiconductor device and that the apparatus further comprises a filter integrated into the semiconductor device to filter the output of the sensor. This provides on chip processing with efficient use of silicon. As has been described, the filter may be provided by an operational transconductance amplifier and a capacitor. In this example, the capacitor is in parallel with the amplifier output to provide a low pass filter and there may be variable bias means operable to set the transconductance of the amplifier.

### Approximation of frequency weighted $\omega^{0.5}$ filter

The frequency weighted filter at the pixel level can also be realized by an alternative configuration of the OTA-C technique (Fig. 5c). The frequency weighted filter comprises a capacitor 48 and an OTA 49 with an external DC bias voltage 50 applied to the positive input of the OTA to provide an appropriate operating point and level shift. This OTA-C is actually a high pass filter, and by setting an appropriate bias current level, the cut-off frequency can be set at ~10 kHz. Although this is not the precise $\omega^{0.5}$ filter required in laser Doppler blood flowmetry to provide a linear retationship between velocity and flow, it does provide a useful approximation when minimizing silicon area is essential. The measured and simulated frequency responses of the frequency weighted filter are shown in Fig. 5d.

It can be understood from the above description that in this example, the sensor is a semiconductor device and that the apparatus further comprises a filter integrated into the semiconductor device to filter the output of the sensor. This provides on chip processing with efficient use of silicon. As has been described, the filter may be provided by an operational transconductance amplifier and a capacitor. In this example, the capacitor is in series with the inverting input of the amplifier, to provide a high pass filter. In this example, there may be variable bias means operable to apply a DC bias current to set the cutoff frequency of the filter to provide an approximate $\dot{\omega}^{0.5}$ filter with the expected Doppler signal frequencies from a subject to be imaged. The cut-off frequency may be approximately 10 kHz.

### Equivalence of 'absolute and average' and 'square and average' processing

Replacing 'square and average' processing with 'absolute and average' processing is advantageous in terms of the amount of silicon area used. The following derivation demonstrates the equivalence of these approaches.

Squaring the band pass and frequency weighted filter output and then averaging allows the concentration and the flow to be obtained.

$$Concentration = \int_{20\,Hz}^{20\,KHz} P(\omega)d\omega = \frac{\sum_{n=0}^{M-1}(BP(n))^2}{M} \qquad (6)$$

$$Flow = \int_{20\,Hz}^{20\,KHz} \omega P(\omega)d\omega = \frac{\sum_{n=0}^{M-1}(FW(n))^2}{M} \qquad (7)$$

Where $P(\omega)$ is the power of the AC component of the Doppler; BP (n) and FW (n) are the outputs of band pass filter and frequency weighted filter at time n respectively; and M is the number of points used when averaging.

The square and average circuit takes up a significant amount of silicon space because of the multiplication process and the summation of large numbers. For example, a logic gate count study has been carried out on the "square & average" (over 512 points) design for three filters using Xilinx FPGA ISE7.1 software and the results showed an equivalent gate count of 13,728. As the gate density of the standard $0.35\mu m$ CMOS process is 15,000 gates/mm$^2$, the "square & average" design will take up a silicon area of 0.92mm$^2$.

Let us now take the "absolute" of the output from the concentration and flow filters and then average (i.e. integrate) these signals. We arrive at;

$$Concentration = \int_{20Hz}^{20KHz} |V(\omega)|d\omega = \frac{\sum_{n=0}^{M-1}|BP(n)|}{M} \tag{8}$$

$$Flow = \int_{20Hz}^{20KHz} |\omega^{1/2}V(\omega)| \, d\omega = \frac{\sum_{n=0}^{M-1}|FW(n)|}{M} \tag{9}$$

For a Gaussian signal distribution, the variance of the signal distribution is defined as:

$$\sigma^2 = \frac{\sum_{i=1}^{M}(x_i - \mu)^2}{M}$$
$$= \frac{\sum_{i=1}^{M}x_i^2 + \sum_{i=1}^{M}\mu^2 - 2\mu\sum_{i=1}^{M}x_i}{M} \tag{10}$$
$$= \frac{\sum_{i=1}^{M}x_i^2}{M} + \mu^2 - 2\mu^2$$
$$= \frac{\sum_{i=1}^{M}x_i^2}{M} - \mu^2$$

As the output of the band pass filter and frequency weighted filter follow a Gaussian distribution with zero mean, "square and average" effectively works out the variance.

$$Concentration = \frac{\sum_{n=0}^{M-1}(BP(n))^2}{M} = \sigma^2(BP) \tag{11}$$

$$Flow = \frac{\sum_{n=0}^{M-1}(FW(n))^2}{M} = \sigma^2(FW) \tag{12}$$

On the other hand, "absolute and average" process takes the absolute of the filtered output followed by averaging. Statistically the process can be described as follows.

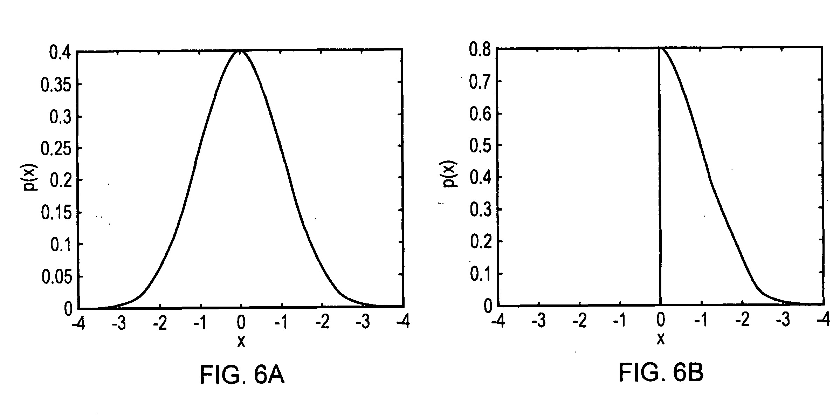

The probability density function of a standard normal distribution p(x) is shown in Figure 6a. After the 'absolute' process, the distribution p'(x) is shown in Figure 6b where p(x) and p'(x) are given by;

$$p(x) = \frac{1}{\sigma\sqrt{2\pi}} e^{-\frac{1}{2}\left(\frac{x-\mu}{\sigma}\right)^2} \qquad (-\infty \le x \le \infty)$$

$$(13)$$

$$p'(x) = \frac{2}{\sigma\sqrt{2\pi}} e^{-\frac{1}{2}\left(\frac{x-\mu}{\sigma}\right)^2} \qquad (0 \le x \le \infty)$$

$$(14)$$

Note that the magnitude of p'(x) is double that of p(x) as the range is halved.

The average of the normal distribution after the 'absolute' process is defined as:

$$\mu' = \int_0^\infty x p'(x)dx$$

$$= \int_0^\infty x \frac{2}{\sigma\sqrt{2\pi}} e^{-\frac{1}{2}(\frac{x-\mu}{\sigma})^2} dx$$

$$(15)$$

Where $\square'$ is the final output of the absolute and average process.

Let $\dfrac{x-\mu}{\sigma} = X$, and $dX = \dfrac{1}{\sigma}dx$,

$$\mu' = \int_0^\infty (\sigma X + \mu) \frac{2}{\sigma\sqrt{2\pi}} e^{-\frac{1}{2}X^2} \sigma dX$$

$$= \frac{2\sigma}{\sqrt{2\pi}} \int_0^\infty X e^{-\frac{1}{2}X^2} dX + \frac{2\mu}{\sqrt{2\pi}} \int_0^\infty e^{-\frac{1}{2}X^2} dX$$

As $\mu = 0$ (for the "band pass" based system) then:

$$\mu' = \frac{2\sigma}{\sqrt{2\pi}} \int_0^\infty X e^{-\frac{1}{2}X^2} dX$$

Let $U = \dfrac{X}{\sqrt{2}}$, then $dU = \dfrac{1}{\sqrt{2}}dX$,

$$\mu^{\cdot} = \frac{2\sigma}{\sqrt{2\pi}} \int_0^\infty \sqrt{2}Ue^{-U^2}\sqrt{2}dU$$

$$= \frac{2\sqrt{2}\sigma}{\sqrt{\pi}} \int_0^\infty Ue^{-U^2}dU \qquad\qquad (16)$$

$$= \frac{-\sqrt{2}\sigma}{\sqrt{\pi}} e^{-U^2}\Big|_0^\infty$$

$$= \frac{\sqrt{2}}{\sqrt{\pi}}\sigma$$

If the mean is not zero then the Equation 16 contains an offset which can be calibrated out.

Finally. as can be seen from Equation 16, the "absolute and average" of the Gaussian distribution with zero mean is proportional to the standard deviation of the Gaussian distribution, by a scaling factor of $\frac{\sqrt{2}}{\sqrt{\pi}}$. As a result,

the square of the "absolute and average" is also proportional to the variance.

$$\mu^{\cdot 2} = \frac{2}{\pi}\sigma^2$$

$$(17)$$

The concentration and flow is then defined as

$$\left(Concentration^{\cdot}\right)^2 = \mu^{\cdot 2} = \left(\frac{\sum_{n=0}^{M}|BP(n)|}{M}\right)^2 = \frac{2}{\pi}\sigma^2(BP) \qquad\qquad (18)$$

$$\left(Flow^{\cdot}\right)^2 = \mu^{\cdot 2} = \left(\frac{\sum_{n=0}^{M}|FW(n)|}{M}\right)^2 = \frac{2}{\pi}\sigma^2(FW) \qquad\qquad (19)$$

By comparing equations 18 and 19 to equations 11 and 12, it can be seen that "absolute and average" followed by square is proportional to the "square and average", by a factor of $\frac{2}{\pi}$.

It should be noted that this scaling factor is valid for Gaussian data. We have analyzed Doppler from a laser Doppler blood flow imager and have seen this to be the case. The scaling factor for other PDFs can also be calculated. For example, for a PDF of the form

$$p(x) = \begin{cases} \dfrac{1}{a} + \dfrac{1}{a^2}x & (-a \le x \le 0) \\ \dfrac{1}{a} - \dfrac{1}{a^2}x & (0 < x \le a) \end{cases}$$

Here, the 'absolute and average' is proportional to the 'square and average' by a scaling factor is 2/3.

The "absolute and average" of a DC channel is quite straight forward. As the low pass filter output is always positive, the "absolute and averaging" is simply an averaging circuit, which works out DC voltage over the number of averaged data.

$$DC' = \frac{\sum_{n=0}^{M-1} |LP(n)|}{M} \qquad (20)$$

The squaring in Equations 18 and 19 on BP(n) and FW(n) can be performed on a PC. Since only one square process is required for one "absolute and average" output, the processing time is greatly reduced compared to the "square and average", which requires one square process for every input.

Furthermore, as the square term involves multiplication while absolute term only requires the truncation of the signed bit. Thus the gate count of the "absolute & average" design (1,461 gates over 512 points) is much less than that of the "square & average" design (13,728 gates over 512 points).

Therefore replacing square and average with absolute and average electronics allows a considerable saving in silicon area.

Accordingly, this example proposes that the apparatus further comprises a band pass filter and a frequency weighted filter, each operable to filter the output sensor, and processing means operable to provide average values over time of the absolute values of the filter outputs.

The sensor element, the band pass filter and the frequency weighted filter may each be semiconductor devices, so that they may be integrated into the semiconductor device in order to provide on chip processing with efficient use of silicon.

### *Parametric fitting of models of laser Doppler frequency spectra to obtain estimates of blood flow*

When processing is performed off-chip, the filters are often implemented through first taking a fast Fourier transform (FFT) over a large number of points (e.g. 512, 1024 points). The filters are then implemented by processing the FFT. On-chip, however, this would consume a large silicon area. The processing can be simplified by extracting less data in the frequency domain, fitting to the data in the frequency domain and then applying the filters.

The form of the data in the frequency domain is known to be [27]

$$S(v) = k \exp\left(-\frac{v}{\langle v \rangle}\right)$$

Less points in the frequency can be obtained by implementing fewer points in an FFT, or implementing dicrete band-pass filters or lock-in amplifiers.

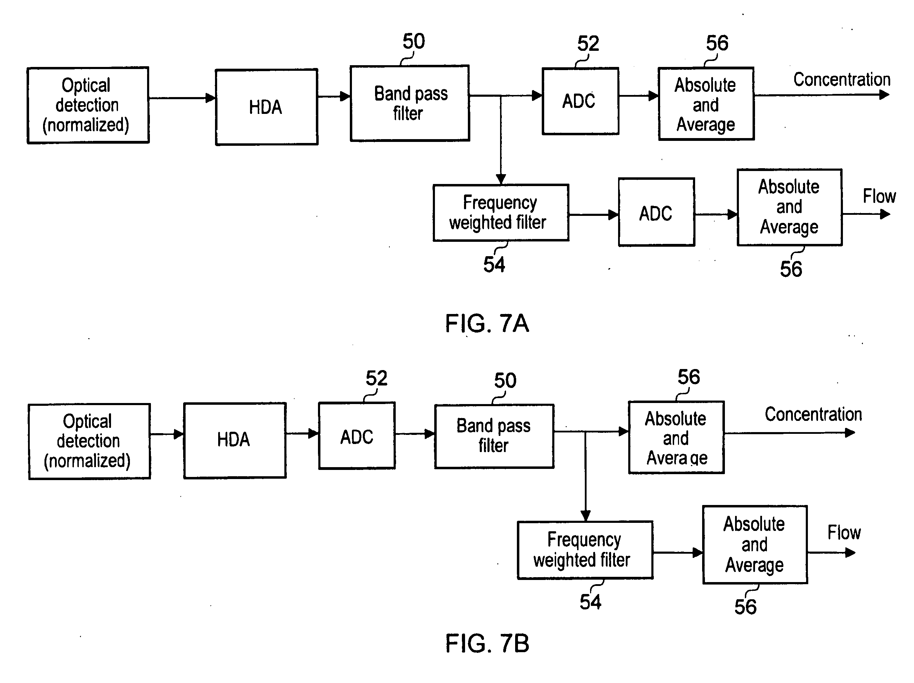

When using the absolute and average process the new block diagram for the processing electronics is shown in figure 7 utilizing a band pass fitter 50. Here, the position of the analogue to digital converter 52 is flexible, depending on whether the concentration and flow processing are implemented in either analogue or digital electronics. For example when the design is mainly analogue (see figure 7(a)) then the ADC is placed after the band pass filter 50 and frequency weighted filter 54. Subsequent to this the absolute and average is implemented digitally at 56. Alternatively figure 7(b) shows the block diagram of the new processing when the design is mainly digital. In this case the band pass filter 50 and frequency weighted filter 54 are implemented in digital along with the absolute and average processing 56.

Instead of using the logarithmic pixel but utilising the integrating pixel it would be apparent to one skilled in the art that this is a digital pixel when combined with a comparator and a counter. However, in this realization a distinct technical advantage is that no ADC is necessary and hence the bandpass filter, frequency weighted filter and absolute and average can .all be implemented digitally thereby resulting in a considerable saving in silicon integrated circuit area.

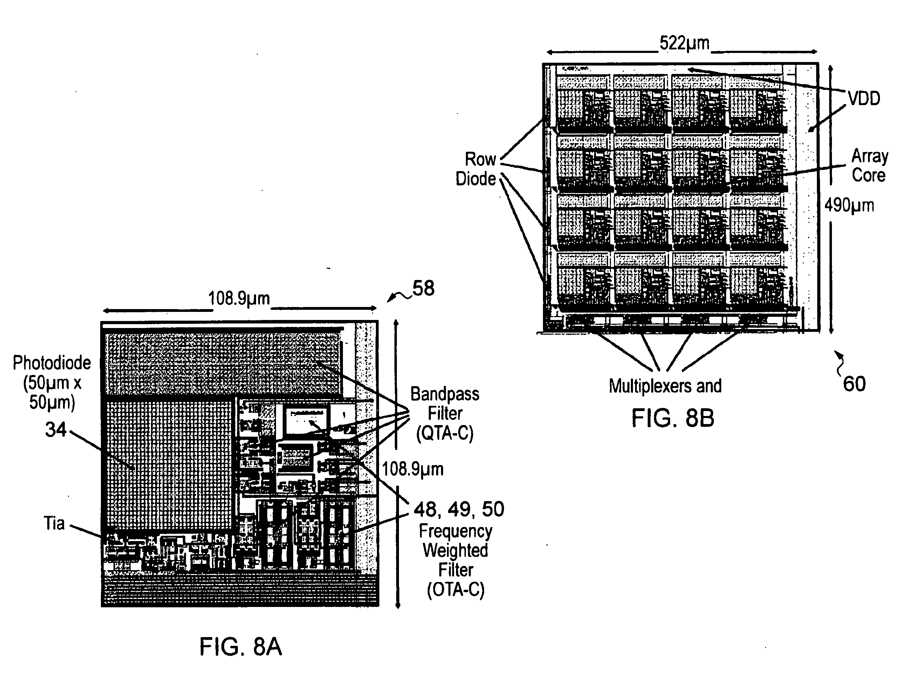

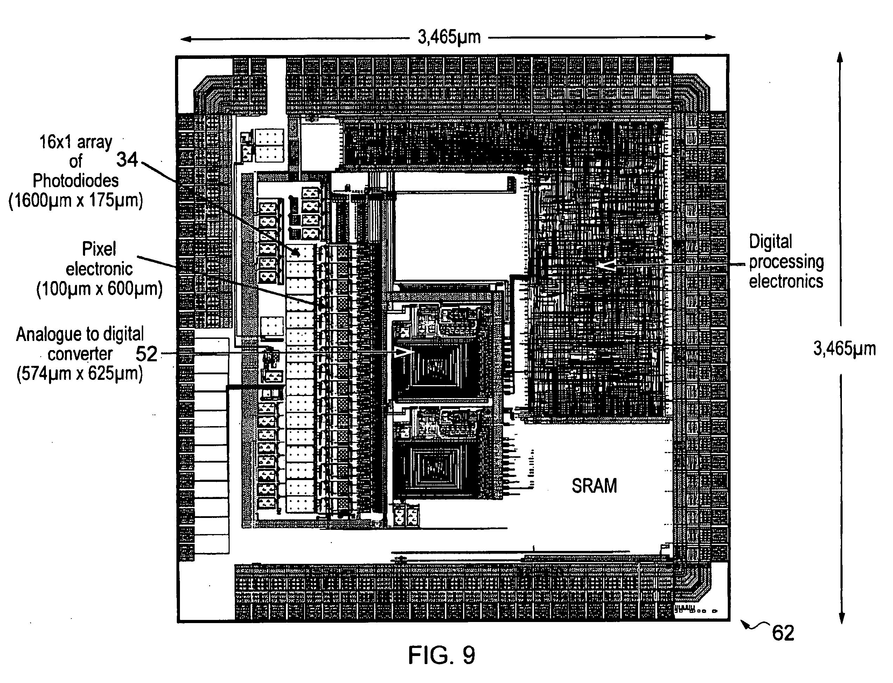

CMOS sensor chips implemented using one or more of these approaches are shown in figures 8 and 9 in chips of typical size 3mm x 3mm. However, it will be appreciated that any chip size, subject to yield and light power restictions, could be deployed.

Processing of the sensor outputs may be performed digitally, by analogue techniques, or by a mixture of digital and analogue techniques. Processing of the sensor outputs may be performed separately for each pixel or for groups of pixels, such as a row, column or other subset of an array of pixels.

Fig. 8a shows a single pixel 58 of an array. Fig. 8b illustrates a 4x4 array 60. Fig. 9 illustrates a 16x1 linear photodiode array 62 comprising log pixel, HDA and anti-aliasing filter at the pixel level. In this example, the digital electronics contains frequency weighted, band pass and low-pass filters, along with absolute and average circuits, as disclosed above. Alternatively, analogue techniques could be used.

To conclude, it will be appreciated that;

- Although we have presented a complete system, one or more of the innovative steps can be combined to produce an improved laser Doppler on-chip processing system
- The sensor could be applied in any sort of imaging system e.g camera, endoscope
- While CMOS fabrication is discussed other processes such as Indium Gallium Arsenide (InGaAs), Mercury Cadmium Teluride (HgCdTe) for example can be used to change the wavelength sensitivity range.
- The processing can be used for either 2D arrays of photodiodes, 1 D arrays of photodiodes or single photodiodes on chip. For 1 D arrays and single pixel imaging this would mean that scanning would need to be performed to build up an image.

It is apparent from the above description that in the use of CMOS sensors in laser Doppler imaging there are considerable advantages in using on-chip processing but to date this has not been feasible due to the silicon area used by the on-chip processing electronics. The examples and embodiments described above make use of various innovative steps in the design of on-chip processing. Namely:

- The use of a logarithmic pixel or similar non-linear detector to perform normalization and hence remove the need for on-chip division and the requirement of a DC light channel
- The use of a combined integrating pixel and digital counter to perform normalisation
- The use of a hysteretic differentiator amplifier or amplifier with a similar response to amplify the AC and leave the DC unaffected. This reduces the number of bit resolution of an on-chip analogue to digital converter and reduces silicon area.
- The use of operational transconductance filters for efficient implementation of filters
- The use of a frequency weighted high pass filter to replace the $\omega^{0.5}$ filter conventionally used
- The use of absolute and average processing electronics to replace the square and average conventionally used.
- Parametric fitting of models of laser Doppler frequency spectra to obtain estimates of blood flow

**References**

1) LE Drain, The laser Doppler technique, Wiley 1980.

2) F Durst, a Melling, JH Melling, Principles and Practice of laser Doppler anemometry, Academic Press 1981.

3) BKA Ngoi, K Venkatakrishnan, Opt. Eng. 39:2995-3000, 2000.

4) EB Li, AK Tieu, WYD Yuen, Optics and Lasers in Engineering, 35:41-49, 2001.

5) RW Ainsworth, SJ Thorpe, RJ Manners, Int. J. Heat and Fluid Flow 18:116-130, 1997.

6) J Kuhlman, L Burton, T Scarberry, Meas. Sci. Tech. 13:1154-1162, 2002.

7) MV Aguanno, F Lakestani, MP Whelan, MJ Connelly, Proc SPIE 5251:304-312,2004.

8) MV Aguanno, MP Whelan, MJ Connelly, Proc SPIE 4827:123-132, 2002.

9) A Kimachi, S Ando. In Optoelectronic Integrated Circuits IV, San Jose, Ca, USA, Proceedings of SPIE Vol. 3950, 2000.

10)F Chen, WD Luo, M Dale, A Petniunas, P Harwood and GM Brown, Opt. and Las. in Eng., 40:459-485, 2003.

11) J Leval, P Picart, JP Boileau, Appl. Opt. 44:5763-5772, 2005

12)HE Albrecht, M Borys, N Damaschke, C Tropea, Laser Doppler and Phase Doppler Techniques, Springer 2003.

13)UK Application Number 0714192.2, Apparatus for full field flow and vibration imaging (filed on 20 July 2007)

14) Essex, T. J. H., and Byrne, P.O. (1991). "A laser Doppler scanner for imaging blood flow in skin." J. Biomed.

Eng 13: 189-194.

15) Briers, J. D. (2001). "Laser Doppler, speckle and related techniques for blood perfusion mapping and imaging." Physiol. Meas. 22: R35-R66.

16)GB2330719 *Apparatus for Imaging Microvascular Blood Flow,* 2000.

17)GB2413022 *Laser Doppler imaging using a two dimensional random access high pixel readout rate image sensor,* 2006

18)Belcaro, G. V., Hoffmann, U., Bollinger A., and Nicolaides, A. N. (1994). Laser Doppler, Med-Orion.

19)A. Moini, Vision Chips, (Kluwer Academic Publishers, 2000).

20)P.E. Allen and D.R. Holberg, CMOS Analog Circuit Design, (Oxford University Press, 1987).

21)Mead, C. A. (1989). Analog VLSI and neural systems, Addison-Wesley publishing company, Inc.

22)P Dmchowski, CMOS modulated light camera, PhD thesis, University of Nottingham 2006.

23)F. Irons, Active Filters (Artech House, 2005).

24)R. L. Geiger, P. E. Allen, and N. R. Strader, VLSI Design Techniques for Analog and Digital Circuits (McGraw-Hill,1990).

25)R. L. Geiger and E. Sánchez-Sinencio, "Active Filter Design Using Operational Transconductance Amplifiers: A Tutorial", IEEE Circuits and Devices Magazine, 1, 20 (1985).

26)Geiger, R. L., and Sanchez-Sinencio, E. (1985). "Active Filter Design using Operational Transconductance Amplifiers: A Tutorial." IEEE Circuits and Devices Magazine 1:20-32.

27)Serov A and Lasser T, "Combined laser Doppler and laser speckle imaging for real-time blood blow measurements," Proc. SPIE 6094 609406-8 (2006).

28) Pui B H, Hayes-Gill B R, Clark M, Somekh M G and See C W "Integration of a Photodiode Array and Centroid Processing on a Single CMOS Chip for a Real Time Shack Hartmann Wavefront Sensor" ,IEEE SENSORS JOURNAL 4 (6): 787-794 DEC 2004

29)Quan Gu, University of Nottingham PhD thesis, "An analogue circuit design for laser Doppler blood flow measurements", 2007, School of Electrical and Electronic Engineering, Section 4.2 Figure 4.7

30) US Patent, James Woolhouse "Measurement of sperm motility", US4601578, June 1986

31)Frank Comhaire et al "Opinion: Objective semen abnalysis: has the target been reached?" Journal of Human Reproduction, vol 7 , no. 2, pp 237-241, 1992.

## Claims

1. Apparatus comprising:

   at least one electromagnetic radiation sensor (12) for producing an output (38) when illuminated by modulated laser light, being a laser Doppler signal;
   and wherein the sensor comprises a photoelectric sensor element (34) being produce a photocurrent (I) when illuminated, and a transistor (36) through which the photocurrent passes **characterised in that** the sensor is configured to provide an output determined by modulated laser light signal illuminating the sensor; and **in that** the transistor is configured to operate in a sub threshold region to provide a DC output voltage which is a logarithmic function of the DC current, and **in that** the transistor is configured to provide an AC sensor output from the photocurrent when the sensor element is illuminated by a modulated laser light signal, the AC output being normalised by the detected DC level.

2. Apparatus according to claim 1, wherein the sensor element (34) is in series with the channel of the transistor (36), the output (38) being taken at the connection between the sensor element and the transistor.

3. Apparatus according to claim 1 or 2, wherein the sensor (12) is a semiconductor device and the transistor (36) is integrated into the semiconductor device.

4. Apparatus according to claim 1, 2 or 3, wherein the apparatus comprises feedback elements (40), provide feedback to the circuit of the sensor element (34) and transistor (36).

5. Apparatus according to any of claims 1 to 4, wherein the photoelectric sensor element (34) is a photodiode.

6. Apparatus according to any preceding claim, wherein the transistor (36) is an MOS transistor.

7. A method comprising:

providing at least one electromagnetic radiation sensor (12) for producing an output (38) when illuminated by modulated laser light; being a laser Doppler signal;

and wherein the sensor comprises a photoelectric sensor element (34) producing a photocurrent (I) when illuminated, and a transistor (36) through which the photocurrent passes, **characterised in that** the sensor, in use, provides an output determined by a modulated laser light signal illuminating the sensor; and **in that** the transistor operates in a sub threshold region to provide a DC output voltage which is a logarithmic function of the DC current, and **in that** the transistor provides an AC sensor output from the photocurrent when the sensor element is illuminated by a modulated laser light signal, the AC output being normalised by the detected DC level.

8. A method according to claim 7, wherein the sensor element (34) is in series with the channel of the transistor (36), the output (38) being taken at the connection between the sensor element and the transistor.

9. A method according to claim 7 or 8, wherein feedback elements (40) are provided which, in use, provide feedback to the circuit of the sensor element (34) and transistor (36).

10. A method according to any of claims 7 to 9, wherein the photoelectric sensor element (34) is a photodiode.

11. A method according to any of claims 7 to 10, wherein the transistor (36) is an MOS transistor.

**Patentansprüche**

1. Vorrichtung, welche aufweist:

mindestens einen Sensor (12) für elektromagnetische Strahlung zum Erzeugen einer Ausgabe (38), wenn er durch moduliertes Laserlicht bestrahlt wird, bei dem es sich um ein Laser-Dopplersignal handelt; und wobei der Sensor ein photoelektrisches Sensorelement (34), das zur Erzeugung eines Photostroms (I) ausgelegt ist, wenn es bestrahlt wird, und einen Transistor (36) umfasst, durch den der Photostrom hindurchtritt, **dadurch gekennzeichnet, dass** der Sensor dazu ausgelegt ist, eine Ausgabe zu erzeugen, die durch ein den Sensor bestrahlendes moduliertes Laserlicht-Signal bestimmt wird; und dass der Transistor für einen Unter-Schwellwert-Bereich ausgelegt ist, um eine Ausgabe-Gleichspannung zu erzeugen, die eine logarithmische Funktion des Gleichstroms ist; und dass der Transistor dazu ausgelegt ist, um von dem Photostrom eine AC-Sensorausgabe zu erzeugen, wenn das Sensorelement durch ein moduliertes Laserlicht-Signal bestrahlt wird, wobei die AC-Ausgabe durch den detektierten DC-Pegel normalisiert ist.

2. Vorrichtung nach Anspruch 1, **dadurch gekennzeichnet, dass** das Sensorelement (34) mit dem Kanal des Transistors (36) in Serie ist, wobei die Ausgabe (38) an der Verbindung zwischen dem Sensorelement und dem Transistor erfolgt.

3. Vorrichtung nach Anspruch 1 oder 2, **dadurch gekennzeichnet, dass** der Sensor (12) ein Halbleiter-Bauelement ist und der Transistor (36) in das Halbleiter-Bauelement integriert ist.

4. Vorrichtung nach Anspruch 1, 2 oder 3, **dadurch gekennzeichnet, dass** die Vorrichtung Rückkopplung-Elemente (40) aufweist, die eine Rückkopplung zur Schaltung des Sensorelements (34) und des Transistors (36) ermöglichen.

5. Vorrichtung nach einem der Ansprüche 1 bis 4, **dadurch gekennzeichnet, dass** das photoelektrische Sensorelement (34) eine Photodiode ist.

6. Vorrichtung nach einem der vorhergehenden Ansprüche, **dadurch gekennzeichnet, dass** der Transistor ein MOS-Transistor ist.

7. Verfahren, welches aufweist:

Bereitstellen mindestens eines Sensors (12) für elektromagnetische Strahlung zum Erzeugen einer Ausgabe (38), wenn er durch moduliertes Laserlicht bestrahlt wird, bei dem es sich um ein Laser-Dopplersignal handelt; und wobei der Sensor ein photoelektrisches Sensorelement (34) aufweist, das einen Photostrom (I) erzeugt, wenn es bestrahlt wird, und einen Transistor (36) aufweist, durch den der Photostrom hindurchtritt, **dadurch gekennzeichnet, dass** der Sensor im Betrieb eine Ausgabe erzeugt, die durch ein den Sensor bestrahlendes moduliertes Laserlicht-Signal bestimmt wird; und

dass der Transistor in einem Unter-Schwellwert-Bereich betrieben wird, um eine Ausgabe-Gleichspannung zu erzeugen, die eine logarithmische Funktion des Gleichstroms ist; und dass der Transistor von dem Photostrom eine AC-Sensorausgabe erzeugt, wenn das Sensorelement durch ein moduliertes Laserlicht-Signal bestrahlt wird, wobei die AC-Ausgabe durch den detektierten DC-Pegel normalisiert wird.

8. Verfahren nach Anspruch 7, **dadurch gekennzeichnet, dass** das Sensorelement (34) mit dem Kanal des Transistors (36) in Serie ist, wobei die Ausgabe (38) an der Verbindung zwischen dem Sensorelement und dem Transistor erfolgt.

9. Verfahren nach Anspruch 7 oder 8, **dadurch gekennzeichnet, dass** Rückkopplung-Elemente (40) vorgesehen sind, die im Betrieb eine Rückkopplung zur Schaltung des Sensorelements (34) und des Transistors (36) erzeugen.

10. Verfahren nach einem der Ansprüche 7 bis 9, **dadurch gekennzeichnet, dass** das photoelektrische Sensorelement (34) eine Photodiode ist.

11. Verfahren nach einem der Ansprüche 7 bis 10, **dadurch gekennzeichnet, dass** der Transistor ein MOS-Transistor ist.

**Revendications**

1. Appareil comprenant :

au moins un capteur de rayonnement électromagnétique (12) destiné à produire une sortie (38) lorsqu'il est éclairé par une lumière laser modulée, qui est un signal laser Doppler

et dans lequel le capteur comprend un élément capteur photoélectrique (34) qui est conçu pour produire un courant photoélectrique (I) lorsqu'il est éclairé, et un transistor (36) à travers lequel passe le courant photoélectrique, **caractérisé en ce que** le capteur est conçu pour fournir une sortie déterminée par un signal de lumière laser modulée éclairant le capteur ; et **en ce que** le transistor est conçu pour fonctionner dans une région située en dessous d'un seuil pour fournir une tension de sortie en courant continu qui est une fonction logarithmique du courant continu, et **en ce que** le transistor est conçu pour fournir une sortie de capteur en courant alternatif à partir du courant photoélectrique lorsque l'élément capteur est éclairé par un signal de lumière laser modulée, la sortie en courant alternatif étant normalisée par le niveau de courant continu détecté.

2. Appareil selon la revendication 1, dans lequel l'élément capteur (34) est en série avec le canal du transistor (36), la sortie (38) étant prélevée sur la liaison entre l'élément capteur et le transistor.

3. Appareil selon la revendication 1 ou 2, dans lequel le capteur (12) est un dispositif à semi-conducteurs et le transistor (36) est intégré au dispositif à semi-conducteurs.

4. Appareil selon la revendication 1, 2 ou 3, dans lequel l'appareil comprend des éléments de rétroaction (40) qui fournissent une rétroaction au circuit de l'élément capteur (34) et du transistor (36).

5. Appareil selon l'une quelconque des revendications 1 à 4, dans lequel l'élément capteur photoélectrique (34) est une photodiode.

6. Appareil selon l'une quelconque des revendications précédentes, dans lequel le transistor (36) est un transistor MOS.

7. Procédé comprenant l'étape consistant à :

fournir au moins un capteur de rayonnement électromagnétique (12) destiné à produire une sortie (38) lorsqu'il est éclairé par une lumière laser modulée qui est un signal laser Doppler ;
et dans lequel le capteur comprend un élément capteur photoélectrique (34) produisant un courant photoélectrique (I) lorsqu'il est éclairé, et un transistor (36) à travers lequel passe le courant photoélectrique, **caractérisé en ce que** le capteur, en service, fournit une sortie déterminée par un signal de lumière laser modulée éclairant le capteur ; et **en ce que** le transistor fonctionne dans une région située en dessous d'un seuil pour fournir une tension de sortie en courant continu qui est une fonction logarithmique du courant continu, et **en ce que** le transistor fournit une sortie de capteur en courant alternatif à partir du courant photoélectrique lorsque l'élément capteur est éclairé par un signal de lumière laser modulée, la sortie en courant alternatif étant normalisée par le niveau de courant continu détecté.

8. Procédé selon la revendication 7, dans lequel l'élément capteur (34) est en série avec le canal du transistor (36), la sortie (38) étant prélevée sur la liaison entre l'élément capteur et le transistor.

9. Procédé selon la revendication 7 ou 8, dans lequel sont prévus des éléments de rétroaction (40) qui, en service, fournissent une rétroaction au circuit de l'élément capteur (34) et du transistor (36).

10. Procédé selon l'une quelconque des revendications 7 à 9, dans lequel l'élément capteur photoélectrique (34) est une photodiode.

11. Procédé selon l'une quelconque des revendications 7 à 10, dans lequel le transistor (36) est un transistor MOS.

FIG. 1

20

```
       ┌─────────────┐     22            26
       │ Optical     │    ┌──────────┐  ┌─────────┐         ┌─────────────┐
       │ detection   │───▶│ Band pass│─▶│ Divider │────────▶│ Square and  │──▶ Concentration
       │ & linear    │    │ filter   │  │         │         │ Average     │
       │ amplification│    └──────────┘  └─────────┘         └─────────────┘
       └─────────────┘                        │                   30, 32
              │                                │
              │         ┌──────────┐     ┌──────────────┐  ┌─────────────┐
              └────────▶│ Low pass │────▶│ Frequency    │─▶│ Square and  │──▶ Flow
                        │ filter   │     │ weighted     │  │ Average     │
                        └──────────┘     │ filter ω^0.5 │  └─────────────┘
                            24           └──────────────┘
                                               28
```

FIG. 2

FIG. 3B

FIG. 3A

FIG. 4A

FIG. 4B

FIG. 4C

FIG. 5B

FIG. 5D

FIG. 5A

FIG. 5C

EP 2 205 982 B1

FIG. 6A

FIG. 6B

FIG. 7A

FIG. 7B

EP 2 205 982 B1

522μm

VDD

Row Diode

Array Core

490μm

**FIG. 8B**

Multiplexers and

60

108.9μm

58

Photodiode (50μm x 50μm)

34

Bandpass Filter (QTA-C)

108.9μm

Tia

48, 49, 50 Frequency Weighted Filter (OTA-C)

**FIG. 8A**

16x1 array of Photodiodes (1600μm x 175μm) — 34

Pixel electronic (100μm x 600μm)

Analogue to digital converter 52 (574μm x 625μm)

Digital processing electronics

SRAM

3,465μm

3,465μm

62

FIG. 9

EP 2 205 982 B1

FIG. 10A

FIG. 10B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1332718 A
- JP 10221159 A
- GB 0714192 A
- GB 2330719 A
- GB 2413022 A
- US 4601578 A

### Non-patent literature cited in the description

- **DMOCHOWSKI et al.** Camera pixel for coherent detection of modulated light. *ELECTRONICS LETTERS, IEE STEVENAGE, GB,* 28 October 2004, vol. 40 (22), ISSN 0013-5194, 1403-1404
- **LE DRAIN.** The laser Doppler technique,. Wiley, 1980
- **F DURST.** a Melling, JH Melling, Principles and Practice of laser Doppler anemometry,. Academic Press, 1981
- **BKA NGOI ; K VENKATAKRISHNAN.** *Opt. Eng.,* 2000, vol. 39, 2995-3000
- **EB LI ; AK TIEU ; WYD YUEN.** *Optics and Lasers in Engineering,* 2001, vol. 35, 41-49
- **RW AINSWORTH ; SJ THORPE ; RJ MANNERS.** *Int. J. Heat and Fluid Flow,* 1997, vol. 18, 116-130
- **J KUHLMAN ; L BURTON ; T SCARBERRY.** *Meas. Sci. Tech.,* 2002, vol. 13, 1154-1162
- **MV AGUANNO ; F LAKESTANI ; MP WHELAN ; MJ CONNELLY.** *Proc SPIE,* 2004, vol. 5251, 304-312
- **MV AGUANNO ; MP WHELAN ; MJ CONNELLY.** *Proc SPIE,* 2002, vol. 4827, 123-132
- **A KIMACHI ; S ANDO.** In Optoelectronic Integrated Circuits IV. *Proceedings of SPIE,* 2000, vol. 3950
- **F CHEN ; WD LUO ; M DALE ; A PETNIUNAS ; P HARWOOD ; GM BROWN.** *Opt. and Las. in Eng.,* 2003, vol. 40, 459-485
- **J LEVAL ; P PICART ; JP BOILEAU.** *Appl. Opt.,* 2005, vol. 44, 5763-5772
- **HE ALBRECHT ; M BORYS ; N DAMASCHKE ; C TROPEA.** Laser Doppler and Phase Doppler Techniques,. Springer, 2003
- **ESSEX, T. J. H. ; BYRNE, P.O.** A laser Doppler scanner for imaging blood flow in skin. *J. Biomed. Eng,* 1991, vol. 13, 189-194
- **BRIERS, J. D.** Laser Doppler, speckle and related techniques for blood perfusion mapping and imaging. *Physiol. Meas.,* 2001, vol. 22, R35-R66
- **BELCARO, G. V. ; HOFFMANN, U. ; BOLLINGER A. ; NICOLAIDES, A. N.** Laser Doppler. *Med-Orion.,* 1994
- **A. MOINI.** Vision Chips. Kluwer Academic Publishers, 2000
- **P.E. ALLEN ; D.R. HOLBERG.** CMOS Analog Circuit Design. Oxford University Press, 1987
- **MEAD, C. A.** Analog VLSI and neural systems. Addison-Wesley publishing company, Inc, 1989
- CMOS modulated light camera. **P DMCHOWSKI.** PhD thesis. 2006
- **F. IRONS.** Active Filters. Artech House, 2005
- **R. L. GEIGER ; P. E. ALLEN ; N. R. STRADER.** VLSI Design Techniques for Analog and Digital Circuits. McGraw-Hill, 1990
- **R. L. GEIGER ; E. SÁNCHEZ-SINENCIO.** Active Filter Design Using Operational Transconductance Amplifiers: A Tutorial. *IEEE Circuits and Devices Magazine,* 1985, vol. 1, 20
- **GEIGER, R. L ; SANCHEZ-SINENCIO, E.** Active Filter Design using Operational Transconductance Amplifiers: A Tutorial. *IEEE Circuits and Devices Magazine,* 1985, vol. 1, 20-32
- **SEROV A ; LASSER T.** Combined laser Doppler and laser speckle imaging for real-time blood blow measurements. *Proc. SPIE,* 2006, vol. 6094, 609406-8
- **PUI B H ; HAYES-GILL B R ; CLARK M ; SOMEKH M G ; SEE C W.** Integration of a Photodiode Array and Centroid Processing on a Single CMOS Chip for a Real Time Shack Hartmann Wavefront Sensor. *IEEE SENSORS JOURNAL,* December 2004, vol. 4 (6), 787-794
- **QUAN GU.** An analogue circuit design for laser Doppler blood flow measurements. *School of Electrical and Electronic Engineering,* 2007
- **FRANK COMHAIRE et al.** Opinion: Objective semen abnalysis: has the target been reached?. *Journal of Human Reproduction,* 1992, vol. 7 (2), 237-241